# EUROPEAN PATENT APPLICATION

(11) **EP 4 548 839 A1**
(43) Date of publication of application: **07.05.2025**
(21) Application number: 23831632.7
(22) Date of filing: 30.06.2023
(51) Int. Cl.: A61B 5/02

(54) **ADJUSTMENT METHOD FOR PULSE WAVE MEASURING DEVICE, PULSE WAVE MEASURING SYSTEM, AND PULSE WAVE MEASURING DEVICE**

(30) Priority: 30.06.2022 JP 2022106687
(71) Applicant: Minebea Mitsumi Inc., Kitasaku-gun, Nagano 3890293 (JP)
(72) Inventor: HARADA, Koshi, Kitasaku-gun, Nagano 389-0293 (JP); OKA, Hiroyuki, Kitasaku-gun, Nagano 389-0293 (JP)
(74) Representative: Winter, Brandl - Partnerschaft mbB
(86) International application number: PCT/JP2023/024412
(87) International publication number: WO 2024/005193

(57) **Abstract**

The present invention is a method of adjusting a pulse wave measurement device attachable to a user's wrist. The pulse wave measurement device includes: a pulse wave sensor including a strain gauge and configured to acquire pulse wave by measuring radial artery pulse; pressing part for pressing the pulse wave sensor to the radial artery; and output part for outputting a measured value of the pulse wave sensor as digital signal. The adjusting method includes: acquiring pulse wave using pulse wave sensor when the pulse wave measurement device is attached to user's wrist, and outputting measured value thus acquired, as a digital signal; comparing voltage level of the digital signal with a range previously set with respect to maximum voltage level of output part; and adjusting or instructing pressing force on pulse wave sensor in a case where voltage level of the digital signal is outside the previously set range.

## Description

### TECHNICAL FIELD

The present invention relates to a method of adjusting a pulse wave measurement device, a pulse wave measurement system, and a pulse wave measurement device.

### BACKGROUND ART

In recent years, it has been known to acquire a biological signal wave such as a pulse wave or the like containing information regarding a reflected wave due to a blood flow accompanying blood vessel expansion/contraction and vascular resistance, using a vital sensor attached to a wrist.

However, such a vital sensor suffers from occurrence of errors in the signals due to the difference in the pressing force by which the sensor is pressed against the skin.

Thus, PTL 1 discloses calculating the difference between the maximum value and the minimum value of each blood pressure wave of a pulse signal, and continuously adjusting the pressure of a pulse retaining device upward until the difference no longer increases, allowing the blood pressure wave to be sensed at the optimal pressure value.

### CITATION LIST

### PATENT LITERATURE

PTL 1: Japanese Patent Application Laid-Open Publication No. 2021-6291

### SUMMARY OF THE INVENTION

### TECHNICAL PROBLEM

However, in PTL 1, it is necessary to continuously increase the pressure until the maximum value and the minimum value no longer change. Thus, the time taken for the pressure adjustment varies, and the time taken before the main measurement can be performed is uncertain or long.

Thus, in view of the circumstances described above, it is an object of the present invention to provide a method of adjusting a pulse wave measurement device, which can adjust a pressing force to appropriate one while shortening the time taken for the adjustment, and thus can reduce errors.

### SOLUTION TO THE PROBLEM

To solve the problem described above, an embodiment of the present invention is a method of adjusting a pulse wave measurement device attachable to a wrist of a user.

The pulse wave measurement device includes:
a pulse wave sensor including a strain gauge and configured to acquire a pulse wave by measuring a pulse of a radial artery;
a pressing part configured to press the pulse wave sensor to the radial artery; and
an output part configured to output a measured value of the pulse wave sensor as a digital signal.

The adjusting method includes:
acquiring a pulse wave using the pulse wave sensor when the pulse wave measurement device is attached to a wrist of a user, and outputting a measured value thus acquired, as a digital signal;
comparing a voltage level of the digital signal with a range that is previously set with respect to a maximum voltage level of the output part; and
adjusting or instructing a pressing force on the pulse wave sensor, in a case where the voltage level of the digital signal is outside the range that is previously set.

### ADVANTAGEOUS EFFECTS OF THE INVENTION

According to an embodiment, a method of adjusting a pulse wave measurement device can adjust a pressing force to appropriate one while shortening the time taken for the adjustment, and thus can reduce errors.

### BRIEF DESCRIPTION OF THE DRAWINGS

[FIG. 1] FIG. 1 is an overall view of a pulse wave measurement system according to Embodiment 1 of the present invention.
[FIG. 2] FIG. 2 is a side view illustrating an example of a pulse wave measurement device according to Embodiment 1.
[FIG. 3] FIG. 3 is an exploded view of a pulse wave measurement device included in the pulse wave measurement system of FIG. 1.
[FIG. 4] FIG. 4 is a control block diagram of the pulse wave measurement system of Embodiment 1.
[FIG. 5] FIG. 5 is a diagram indicating average values of pulse wave output voltages with respect to change in the belt length.
[FIG. 6] FIG. 6 includes graphs indicating an example of a measured pulse wave, and an acceleration pulse wave calculated from the pulse wave.
[FIG. 7] FIG. 7 is a diagram illustrating inflection points of a typical acceleration pulse wave.
[FIG. 8] FIG. 8 is a graph indicating changes in acceleration pulse waves due to changes in the pressure applied to a pulse wave sensor.
[FIG. 9] FIG. 9 is a diagram illustrating an average value of a pulse wave output level.
[FIG. 10] FIG. 10 includes diagrams indicating inflection points of acceleration pulse waves corresponding to voltage values of average output levels.
[FIG. 11] FIG. 11 is a diagram summing the dispersion of signals due to pressing force changes within a pulse wave measurement range.
[FIG. 12] FIG. 12 is a detailed flowchart of pressure adjustment for pulse wave measurement.
[FIG. 13] FIG. 13 is an overall flowchart of pulse wave measurement.
[FIG. 14] FIG. 14 is a front view of a pulse wave sensor included in a pulse wave measurement device, seen from a pressure detection surface side.
[FIG. 15] FIG. 15 is a cross-sectional view of the pulse wave sensor of FIG. 14.
[FIG. 16] FIG. 16 is a plan view illustrating a strain gauge included in the pulse wave sensor of FIG. 14.
[FIG. 17] FIG. 17 is a cross-sectional view of the strain gauge of FIG. 16.
[FIG. 18] FIG. 18 is a cross-sectional view of a head member of a pulse wave measurement device according to Embodiment 2.
[FIG. 19] FIG. 19 is an overall block diagram of a pulse wave measurement device according to Embodiment 3.
[FIG. 20] FIG. 20 is an overall block diagram of a pulse wave measurement device according to Embodiment 4.

### DETAILED DESCRIPTION OF THE INVENTION

Embodiments for carrying out the present invention will be described below with reference to the drawings. The same components in the drawings will be denoted by the same signs, and duplicate descriptions of such components may be omitted.

### [Embodiment 1]

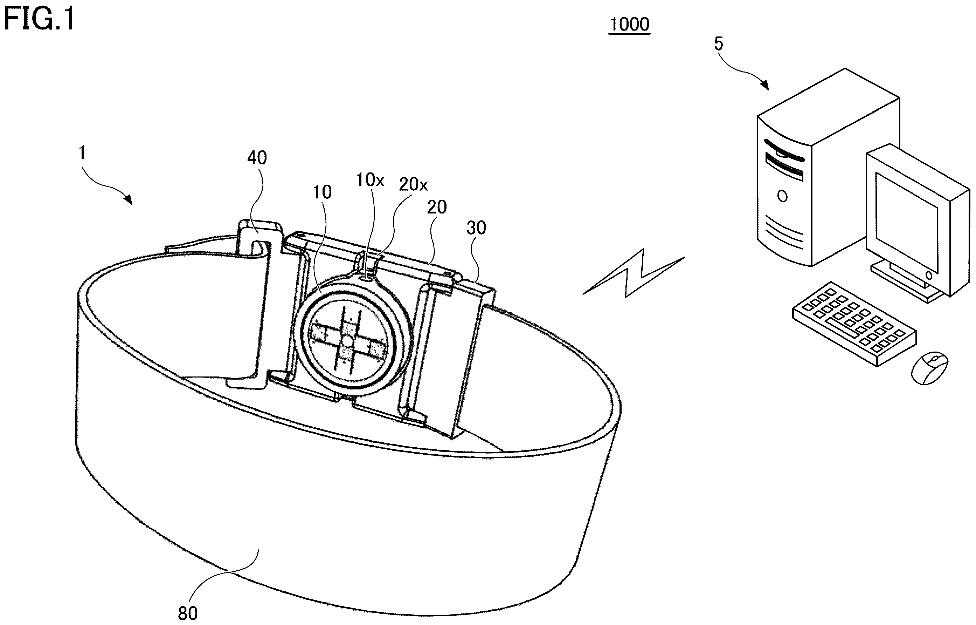

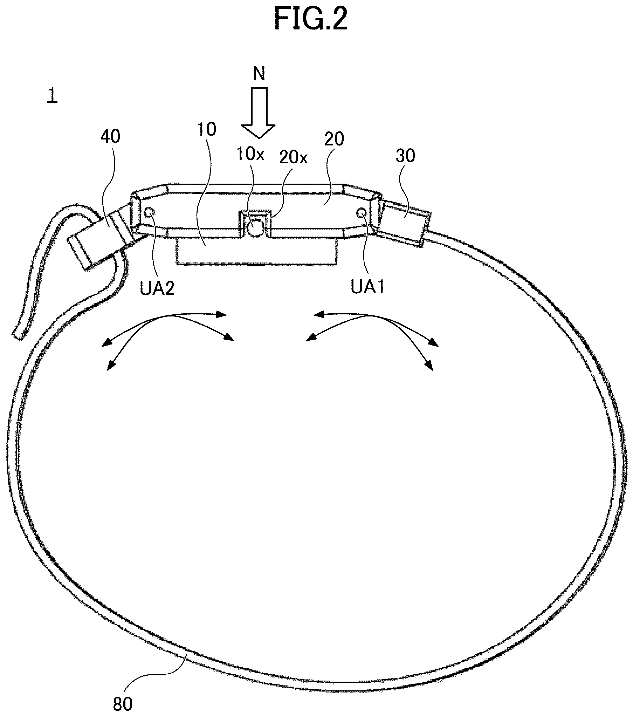

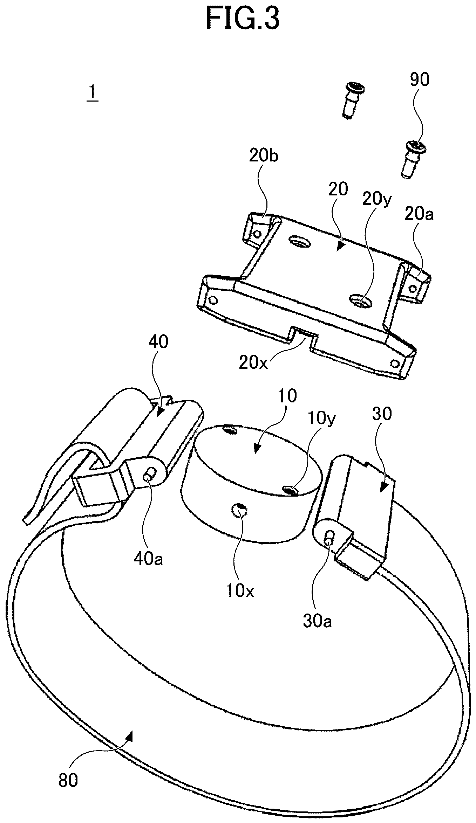

First, a pulse wave measurement system 1000 according to Embodiment 1 will be described with reference to FIGS. 1, 2, and 3. FIG. 1 is an overall view of the pulse wave measurement system 1000 according to Embodiment 1 of the present invention. FIG. 2 is a side view illustrating an example of a pulse wave measurement device 1 according to Embodiment 1. FIG. 3 is an exploded view of the pulse wave measurement device 1 included in the pulse wave measurement system 1000 of FIG. 1.

As illustrated in FIG. 1, the pulse wave measurement system 1000 according to Embodiment 1 includes the pulse wave measurement device 1, and an information processing device 5 that can communicate with the pulse wave measurement device 1.

The pulse wave measurement device 1 is, for example, a vital sensor attached to a wrist of a subject such that a pulse wave sensor 10 is positioned close to the radial artery of the subject. A pulse wave is a waveform capture of changes in the volume of a blood vessel, which occur when the heart pumps blood. The pulse wave measurement device 1 can monitor changes in the volume of a blood vessel.

### (Configuration of pulse wave measurement device according to Embodiment 1)

Referring to FIGS. 1, 2, and 3, the pulse wave measurement device 1 is a wristwatch-type wearable device that can be worn by a subject, and mainly includes the pulse wave sensor 10, a sensor fastener 20, and a belt 80. An arrow N in FIG. 2 indicates a direction normal to a detection surface (a lower surface in FIG. 2) of the pulse wave sensor 10.

The pulse wave sensor 10 is a strain sensor, and is fastened to one side (subject side) of the sensor fastener 20. Specifically, for example, a plurality of screw holes 10y are provided in a back surface side of the pulse wave sensor 10. Moreover, for example, a plurality of insertion holes 20y into which screws are inserted are provided in the sensor fastener 20 so as to penetrate the sensor fastener 20. For example, two screws 90 are inserted into the insertion holes 20y, and their leading ends stick out from the insertion holes 20y to be threadedly engaged with the screw holes 10y, to fasten the pulse wave sensor 10 to the one side of the sensor fastener 20. A positioning hole having a cylindrical shape or the like for positioning the pulse wave sensor 10 may be provided on the one side of the sensor fastener 20.

A through-hole 10x through which a cable for extracting an electric signal from inside the pulse wave sensor 10 may be provided in the side surface of the pulse wave sensor 10. By passing a cable through the through-hole 10x, it is possible to provide wired connection of a signal detected by the pulse wave sensor 10 to an external circuit. For example, a cutout 20x is provided in the sensor fastener 20, and the through-hole 10x is exposed within the cutout 20x.

The belt 80 is an adjusting member, has a band shape for attaching the pulse wave sensor 10 and the sensor fastener 20 to a wrist or the like of the subject, and is configured to be windable about a wrist or the like of the subject from outside. The belt 80 is made of, for example, a resin, a rubber, fabric, or the like, and has flexibility.

One end of the belt 80 is connected to one end side of the sensor fastener 20 uniaxially swingably, and the other end of the belt 80 is connected to the other end side of the sensor fastener 20 uniaxially swingably. More specifically, the one end of the belt 80 is fastened to a belt fastener 30 while being inserted into a groove provided in the belt fastener 30. A projection 30a projecting to both sides of the belt 80 in the width direction is provided on an end of the belt fastener 30 on the sensor fastener 20 side. The projection 30a is inserted into a through-hole of an attachment part 20a provided on the sensor fastener 20, for serving as a shaft UA1.

As a result, as illustrated in FIG. 2, the sensor fastener 20 and the belt fastener 30, to which the one end of the belt 80 is fastened, are connected uniaxially swingably in the directions of arrows about the shaft UA1. The belt fastener 30 and the belt 80 move integrally. That is, the shaft UA1 serves as a shaft for the one end side of the belt 80 to swing.

The other end of the belt 80 is inserted into a through-hole provided in a belt insertion part 40. A projection 40a projecting to both sides of the belt 80 in the width direction is provided on an end of the belt insertion part 40 on the sensor fastener 20 side. The projection 40a is inserted into a through-hole of an attachment part 20b provided on the sensor fastener 20, for serving as a shaft UA2. That is, through the belt insertion part 40, the other end of the belt 80 is connected to the other end side of the sensor fastener 20 swingably about the shaft UA2.

As a result, the sensor fastener 20 and the belt insertion part 40, into which the other end of the belt 80 is inserted, are connected uniaxially swingably in the directions of arrows about the shaft UA2. The belt insertion part 40 and the belt 80 move integrally. That is, the shaft UA2 serves as a shaft for the other end side of the belt 80 to swing. The directions of arrows of FIG. 2 are directions in which the fastening strength of the belt 80 is strengthened or weakened.

By being passed through the through-hole of the belt insertion part 40, the other end of the belt 80 inserted into the through-hole of the belt insertion part 40 can be detachably connected, by, for example, a hook and loop fastener or the like, to part of the outer circumferential surface of the belt 80 that is not inserted into the belt insertion part 40. By changing the longer-direction position of the belt 80, at which the other end of the belt 80 is connected, it is possible to change the fastening strength by which the pulse wave measurement device 1 is attached to the subject.

By the entire body thereof, the pulse wave measurement device 1 can follow the shape of a wrist or the like of the subject, because the sensor fastener 20 and the belt 80 axially swing about the shaft UA1 and the shaft UA2 when the pulse wave measurement device 1 is attached to the subject. Here, by changing the fastening strength of the belt 80, it is possible to press the pulse wave sensor 10 in the direction N. Therefore, it is possible to adjust the degree of close contact between the subject and the pulse wave sensor 10. Thus, a strain generating body 12 side of the pulse wave sensor 10 can be brought into close contact with the radial artery of the subject. In the present embodiment, the sensor fastener 20 serves as a pressing part for pressing the pulse wave sensor 10 to a wrist in response to the belt 80 being fastened.

In FIG. 2, the lower surface of the pulse wave sensor 10 is the detection surface for detecting a pulse wave. The shaft UA1 and the shaft UA2 are positioned on the upper side of the detection surface of the pulse wave sensor 10, i.e., on a side of the detection surface of the pulse wave sensor 10 opposite to the subject. The detection surface of the pulse wave sensor 10 is a surface of the pulse wave sensor 10 that is to be brought into contact with the subject, and is specifically a subject side surface of the strain generating body 12 described below.

The shaft UA1 and the shaft UA2 being positioned on the side of the detection surface of the pulse wave sensor 10 opposite to the subject facilitates pressing the detection surface of the pulse wave sensor 10 to a wrist of the subject through changing the fastening strength of the belt 80. Particularly, it is preferable that the lower surface of the pulse wave sensor 10 projects from the sensor fastener 20 to the subject side. This further facilitates pressing the detection surface of the pulse wave sensor 10 to a wrist of the subject through changing the fastening strength of the belt 80.

It is preferable that the shaft UA1 and the shaft UA2 extend in a direction orthogonal to the longer direction of the belt 80 when the belt 80 is stretched in the left-right direction in FIG. 2 (the orthogonal direction being the depth direction in FIG. 2).

In FIG. 2, i.e., in a side view, it is preferable that a straight line connecting the center of the shaft UA1 and the center of the shaft UA2 is parallel with the detection surface of the pulse wave sensor 10. In this case, the distance between the straight line connecting the center of the shaft UA1 and the center of the shaft UA2 and the detection surface of the pulse wave sensor 10 is, for example, 2 mm or greater and 8 mm or less. Here, being parallel includes the angle formed by the two straight lines being within ±5 degrees. The straight line connecting the center of the shaft UA1 and the center of the shaft UA2 being parallel with the detection surface of the pulse wave sensor 10 makes it possible for the detection surface of the pulse wave sensor 10 to be uniformly pressed to the subject when the belt 80 is fastened.

In a case where the detection surface of the pulse wave sensor 10 is a curved surface that is convex to the subject side, a tangent line on an edge part of the detection surface of the pulse wave sensor 10 that is the closest to the subject is to be considered the detection surface of the pulse wave sensor 10 according to the foregoing description.

In the side view of FIG.2, it is preferable that a straight line that connects the middle point of the straight line connecting the center of the shaft UA1 and the center of the shaft UA2 to the middle point of the detection surface of the pulse wave sensor 10 is perpendicular to the straight line connecting the center of the shaft UA1 and the center of the shaft UA2. Here, being perpendicular includes the angle formed by the two straight lines being within 90±5 degrees. By the straight line that connects the middle point of the straight line connecting the center of the shaft UA1 and the center of the shaft UA2 to the middle point of the detection surface of the pulse wave sensor 10 is perpendicular to the straight line connecting the center of the shaft UA1 and the center of the shaft UA2, it is possible for the detection surface of the pulse wave sensor 10 to be uniformly pressed to the subject when the belt 80 is fastened.

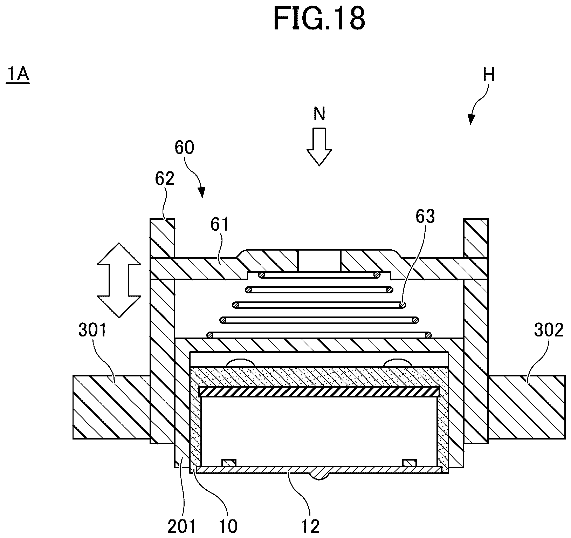

A biasing mechanism for biasing the pulse wave sensor 10 to the subject side may be provided between the sensor fastener 20 and the pulse wave sensor 10. For example, the biasing mechanism may be composed of a spring alone, or a spring and an adjusting member for adjusting the biasing force of the spring, or may be configured in any other manner. The biasing mechanism being provided for biasing the pulse wave sensor 10 to the subject side enables the detection surface of the pulse wave sensor 10 to be pressed to a wrist of the subject stably. A configuration including a spring, of which the biasing force is adjustable, will be described in detail in Embodiment 2 illustrated in FIG. 18.

### (Control block)

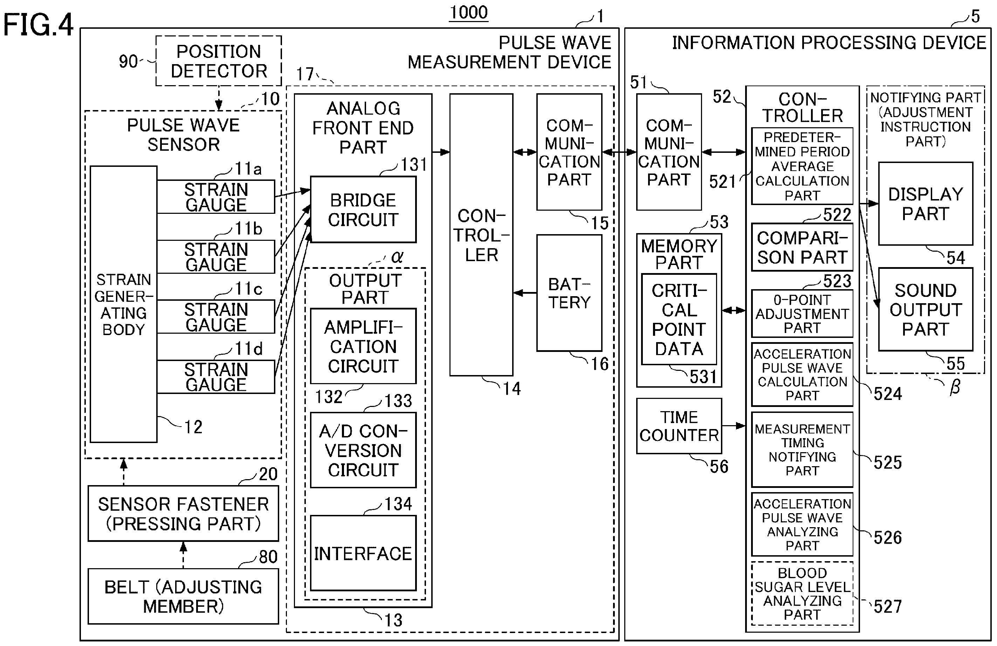

With reference to FIG. 4, the pulse wave measurement device 1 includes the pulse wave sensor 10, a control board 17, the sensor fastener (pressing part) 20, and the belt (adjusting member) 80, as control components. It may further include a position detector 90 configured to detect the position of the pulse wave sensor 10 with respect to the radial artery.

The pulse wave sensor 10 includes the strain generating body 12 and four strain gauges in a housing 100. The control board 17 includes an analog front end part 13, a controller 14, a communication part 15, and a battery 16.

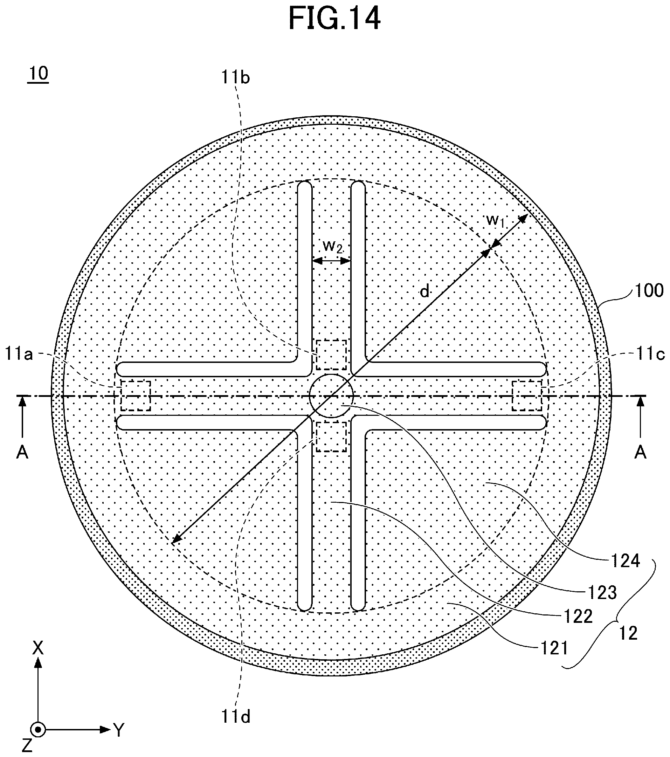

The strain gauges 11a, 11b, 11c, and 11d each include a resistor, of which the resistance value changes in response to a beam portion 122 being elastically deformed due to a load being applied to a load portion 123 (see FIG. 14) of a pulse wave sensor in accordance with a pulse wave of the subject.

The analog front end part (AFE) 13 includes, for example, a bridge circuit 131, an amplification circuit 132, an analog/digital conversion circuit (A/D conversion circuit) 133, an interface 134, and the like. The AFE 13 may include a temperature compensation circuit. In the AFE 13, the amplification circuit 132, the A/D conversion circuit 133, and the interface 134 function as an output part α.

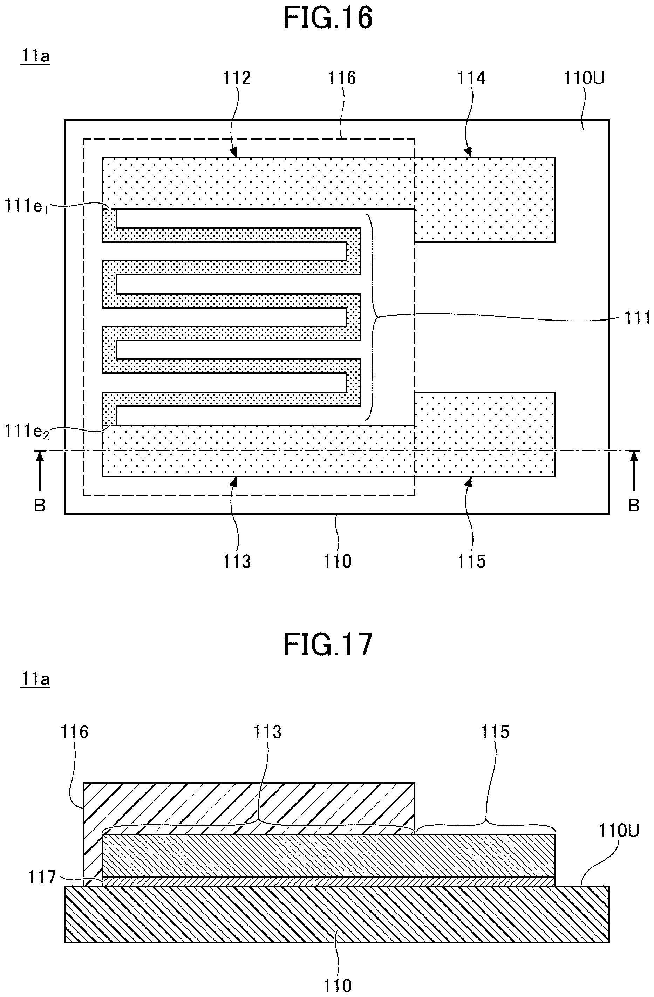

Electrodes 114 and 115 (see FIG. 16), which are the terminals of all of the strain gauges 11a, 11b, 11c, and 11d, are connected to the bridge circuit 131 of the AFE 13, to assemble a full bridge. As a result, a voltage (analog signal) corresponding to changes in the resistance values of the resistors of the four strain gauges 11a, 11b, 11c, and 11d can be output from the bridge circuit 131.

When the resistance values of the plurality of strain gauges 11a, 11b, 11c, and 11d configured in this way change, a consequent change in the positional relationship among the strain gauges 11a, 11b, 11c, and 11d enables pulse wave information to be detected, i.e., a pulse wave to be measured, from the radial artery of the subject with whom they are in close contact. In the present embodiment, an example in which the pulse wave sensor 10 includes four strain gauges is illustrated. However, the number of strain gauges included in the pulse wave sensor 10 is not particularly limited, as long as it is one or more. In a case where the pulse wave sensor 10 includes one strain gauge, "a resistance value of the pulse wave sensor 10" has approximately the same meaning as "a resistance value of the strain gauge".

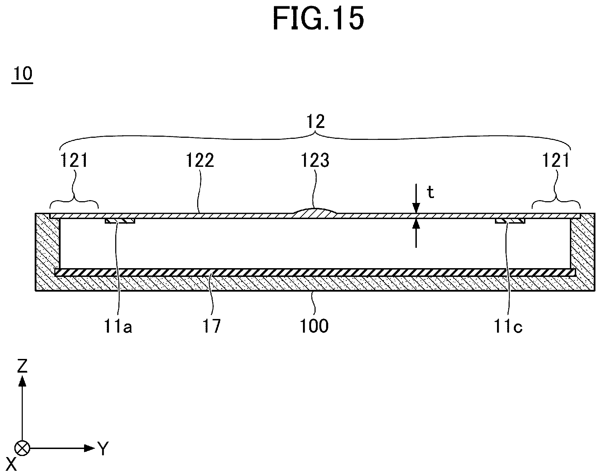

In the pulse wave measurement device 1, the pulse wave sensor 10 including the strain gauges 11a to 11d and the strain generating body 12, and the control board 17 may be provided in the same housing 100 (see FIG. 15), or do not need to be provided in the same housing.

The bridge circuit 131 of the AFE 13 outputs a voltage (analog signal) corresponding to the resistance values of the resistors of the strain gauges 11a to 11d. The voltage output from the bridge circuit 131 is, after being amplified by the amplification circuit 132, converted into a digital signal by the A/D conversion circuit (A/D converter) 133 and sent to the controller 14. In a case where the AFE 13 includes a temperature compensation circuit, a temperature-compensated digital signal is sent to the controller 14.

The controller 14 outputs the digital signal sent from the AFE 13 to the information processing device 5 via the communication part 15. The controller 14 may include a Read Only Memory (ROM), a Random Access Memory (RAM), a main memory, or the like in addition to a calculation part such as a microprocessor, a Central Processing Unit (CPU), a Field Programmable Gate Array (FPGA), an Application Specific Integrated Circuit (ASIC), or the like. For example, the controller 14 is programmed to instruct resistance values to be acquired by the strain gauges 11a to 11d, or to instruct resistance values that are output from the strain gauges 11a to 11d via the AFE 13 to be acquired and sent in accordance with instructions from the information processing device 5. Various functions of the controller 14 can be realized by a program recorded in the ROM or the like being read into the main memory and executed by the calculation part such as the CPU or the like. However, part or the entirety of the controller 14 may be realized only by hardware. Alternatively, the controller 14 may be configured physically by a plurality of devices or the like.

The communication part 15 performs communication between the pulse wave measurement device 1 and the information processing device 5. Specifically, the communication part 15 sends a digital signal resulting from resistance values output from the strain gauges 11a to 11d being converted by the AFE 13 to the information processing device 5. In a case where the pulse wave measurement device 1 operates in accordance with instructions from the information processing device 5, the communication part 15 receives control instructions to the pulse wave sensor 10 from the information processing device 5.

The communication method of the communication part 15 is not particularly limited. For example, the communication part 15 may employ wireless or wired LAN communication methods such as WiFi (registered trademark), Bluetooth (registered trademark), ZigBee (registered trademark), and the like.

The battery 16 is a power source that supplies power to each part of the pulse wave sensor 10 and the control board 17. Instead of including the battery 16, the pulse wave sensor 10 may be supplied with power from an external power source.

With reference to FIG. 4, the information processing device 5 includes at least a communication part 51, a controller 52, and a memory part 53. The information processing device 5 may further include either or both of a display part 54 and a sound output part 55, and a time counter 56.

The communication part 51 acquires a digital signal A/D converted from resistance values, sent out from the communication part 15 of the pulse wave measurement device 1. When the information processing device 5 instructs operations of the pulse wave measurement device 1 such as driving/stopping or the like, the communication part 51 sends control instructions to the pulse wave measurement device 1.

The display part 54 displays letters, images, and the like related to either or both of the contents or the results of processes of the controller 52. The display manner is not particularly limited. The information processing device 5 may include the sound output part 55 together with the display part 54 or instead of the display part 54. The sound output part 55 may output sounds related to either or both of the contents or the results of processes of the controller 52. The display part 54 and the sound output part 55 function as a notifying part β and an adjustment instruction part.

The time counter 56 counts time, and notifies the controller 52 of a pulse wave measurement timing (e.g., 0 minutes past every hour, or a predetermined time (e.g., 12 hours) past the previous measurement) and the like as needed.

The memory part 53 stores information necessary for the operations of the information processing device 5. The memory part 53 stores, for example, critical point data 531. The critical point data 531 is data indicating an output voltage threshold, with which comparison is made in the controller 52 to determine whether or not pressure adjustment of the preliminary adjustment is necessary.

The controller 52 includes, in an executable manner, a predetermined period average calculation part 521, a comparison part 522, a 0-point adjustment part 523, an acceleration pulse wave calculation part 524, a measurement timing notifying part 525, and an acceleration pulse wave analyzing part 526. It may further include a blood sugar level analyzing part 527.

The predetermined period average calculation part 521 and the comparison part 522 correspond to a calculation mechanism for pressure adjustment in the pulse wave measurement of the present invention. Specifically, the predetermined period average calculation part 521 calculates the average value, for a predetermined period, of the voltage level of a digital signal that is A/D-converted from a pulse wave measured immediately after the pulse wave measurement device 1 is attached.

The comparison part 522 compares the average value of the digital signal with the critical point, which is the threshold, and in a case where the average value is less than the critical point, the comparison part 522 notifies via the display part 54 and the sound output part 55 that the pressure should be strengthened.

The 0-point adjustment part 523 performs 0-point adjustment at the initial use.

The acceleration pulse wave calculation part 524 calculates an acceleration pulse wave by calculating the second derivative of the digital signal.

The measurement timing notifying part 525 sends a measurement prompting notification via the display part 54 and the sound output part 55 when a measurement timing has come.

The acceleration pulse wave analyzing part 526 analyzes the health condition of the subject based on the acceleration pulse wave measured this time or the acceleration pulse wave measured in the past.

The blood sugar level analyzing part 527 analyzes the blood sugar level of the subject based on the acceleration pulse wave measured this time or the acceleration pulse wave measured in the past as needed. The blood sugar level analyzing part 527 does not need to be provided.

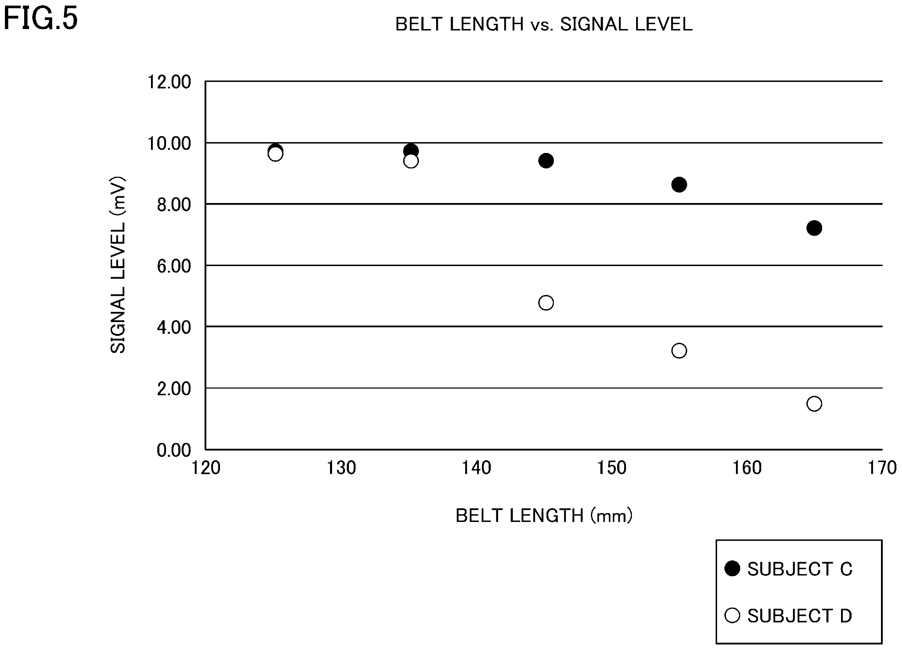

The controller 52 controls the information processing device 5 comprehensively. The controller 22 can be realized by, for example, a CPU, a FPGA, an ASIC, and the like. The controller 52 may include a memory. Various functions of the controller 52 can be realized by a program recorded in a ROM or the like being read into a main memory and executed by the CPU. However, part or the entirety of the controller 52 may be realized only by hardware. Alternatively, the controller 52 may be configured physically by a plurality of devices or the like. The controller 52 determines whether or not pressure adjustment is necessary by comparing a digital signal received from the pulse wave measurement device 1 with the critical point, and calculates an acceleration pulse wave in the main measurement. The contents of the processes of the controller 52 will be described in detail below with reference to FIGS. 5 to 13.

### (Output voltage and belt length)

Here, FIG. 5 is a diagram indicating average values of pulse wave output voltages with respect to change in the belt length. As indicated in FIG. 5, as the belt length is lengthened, the pressing force on the skin applied by the pulse wave sensor 10 decreases, and the signal levels of the digital signals of the pulse waves measured decrease.

However, the girth of a wrist and the flatness of a wrist vary from person to person, so the belt length at which the output voltage becomes low varies from person to person.

Thus, according to the present invention, in a case where the voltage level of a digital signal that is output as a pulse wave measured is compared with a range that is previously set with respect to a maximum voltage level of the output part α, and the voltage level of the digital signal is less than the range, the pressing force on the pulse wave sensor 10 is adjusted, or an instruction for the adjustment is issued.

Thus, it is possible to appropriately adjust the pressure applied to the pulse wave sensor 10 in accordance with the features of a wrist of the person to whom the sensor is attached. For example, in a case where 10 mV is the maximum and the critical point is 4 mV in the example of FIG. 5, the subject C needs no pressure adjustment because the signal level is equal to or higher than the critical point even at a belt length of 165 mm. On the other hand, the subject D receives a pressure adjustment instruction at a belt length of 155 mm or greater because the signal level is lower than the critical point at the belt length of 155 mm.

In this way, the pressure applied to the pulse wave sensor 10 can be confirmed by the level of the output signal that is output from the pulse wave sensor 10 itself. Thus, it is possible to adjust the pressure based on the level of the output signal and to bring the output signal level to within an appropriate range without a device such as a pressure gauge. Thus, it is possible to suppress signal errors that may result from the pressing force difference due to the arm girth that is different depending on the subject, or from changes in the pressing force in repetitive measurements on the same subject, or the like.

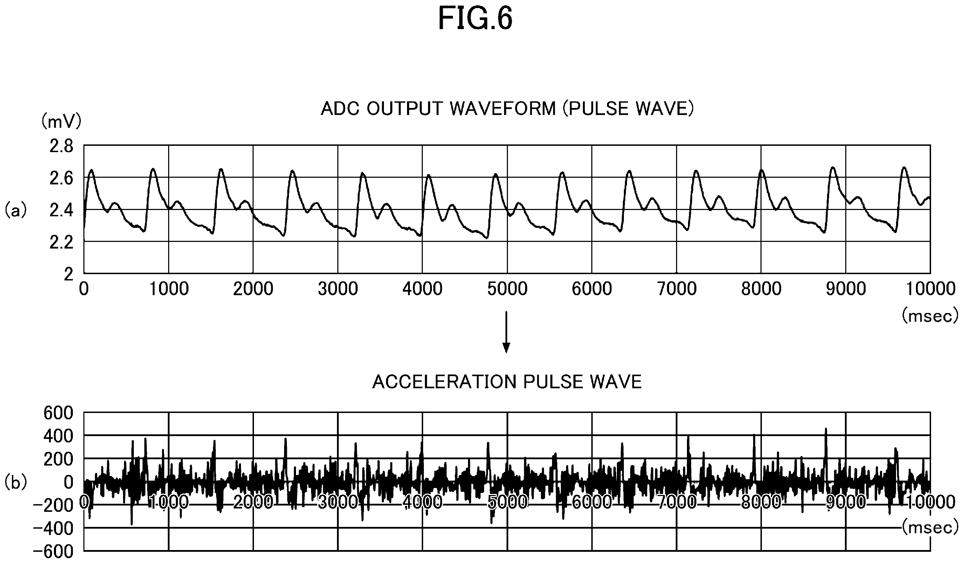

FIG. 6 indicates graphs of examples of a pulse wave measured, and of an acceleration pulse wave calculated from the pulse wave. In FIG. 6, (a) indicates a digital output waveform that is A/D-converted from a pulse wave, and (b) indicates the waveform of an acceleration pulse wave.

The acceleration pulse wave calculation part 524 of the information processing device 5 calculates an acceleration pulse wave by calculating the second derivative of the digital signal A/D-converted from the pulse wave after noise removal, and smoothing/standardization. Since the digital pulse wave that is measured and A/D-converted by the pulse wave measurement device 1 corresponds to the heartbeat finger plethysmogram, the acceleration pulse wave corresponds to a waveform obtained by calculating the second derivative of the finger plethysmogram that is the original waveform. In general, pulse conditions can be analyzed based on the acceleration pulse wave form.

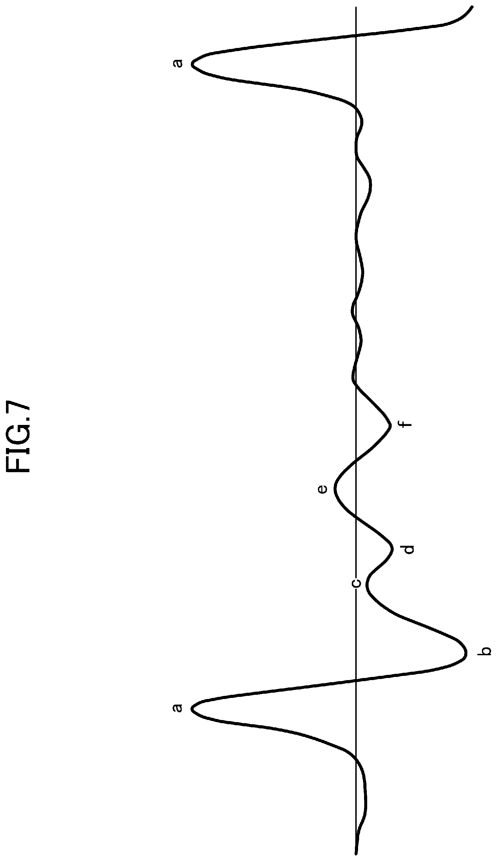

FIG. 7 indicates a diagram illustrating inflection points of a typical acceleration pulse wave.

The inflection point a (a wave) is the first inflection point in the cycle, is referred to as a protosystole positive wave, and indicates the maximum point on the blood vessel expansion speed changing rate. The inflection point b (b wave) is the second inflection point in the cycle, is referred to as a protosystole negative wave, and is shallow (has a small absolute value) when the arterial distensibility is poor. The a wave and the b wave are systolic anterior components, and reflect a drive pressure wave that is generated by blood ejection.

The inflection point c is the third inflection point in the cycle, is referred to as a mid-systole re-elevation wave, and is a wave reflected due to vascular resistance. The inflection point d is the fourth inflection point in the cycle, is referred to as a late systolic re-descent wave, and is the end point of the wave reflected due to vascular resistance. The higher the vascular resistance, the deeper the inflection point (the greater the absolute value).

The inflection point e is the fifth inflection point in the cycle, and is also referred to as a protodiastole positive wave (e wave). The inflection point f is the sixth inflection point in the cycle, and is also referred to as a protodiastole negative wave (f wave).

The c wave and the d wave are systolic posterior components, which reflect a reflected pressure wave that is the drive pressure wave that has propagated to the periphery and returned by being reflected. Here, it is commonly known that the systolic posterior components (c wave and d wave) may rise relatively to the systolic anterior components (a wave and b wave) through increase in the wave reflected due to aging, arteriosclerosis, high blood pressure, or the like. Particularly, the depth of the d wave deepens relatively to the depth of the b wave along with increase in the vascular age. Therefore, by analyzing the inflection points on the acceleration pulse wave, it is possible to know the vascular age or the condition of the artery.

In order to calculate and analyze an acceleration pulse wave, as a premise, it is necessary to measure a pulse wave that is the original waveform of the acceleration pulse wave under appropriate conditions.

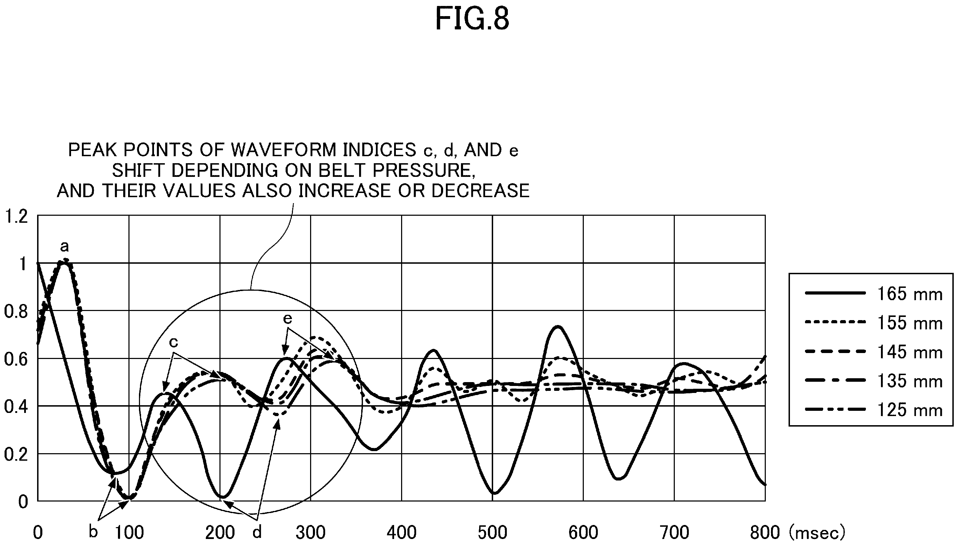

Here, FIG. 8 illustrates a graph indicating changes of acceleration pulse waves due to changes in the pressure applied to the pulse wave sensor 10. Specifically, FIG. 8 is a graph indicating acceleration pulse waves in a case of measuring pulse waves of the same subject, who is a male in his fifties, while changing the belt pressure.

As described above, in the pulse wave measurement device according to Embodiment 1, the pressing force by which the detection surface of the pulse wave sensor is pressed on a wrist of the subject changes in response to changes in the belt length. For this reason, along with the variation of the waveforms of pulse wave digital signals depending on the belt pressure as is known from FIG. 5, the amplitude direction positions and timings of the c wave, the d wave, and the e wave, which are waveform indices, also vary in the acceleration pulse waves calculated from the digital signals as illustrated in FIG. 8.

In the example of FIG. 8, particularly in a case of a belt length of 165 mm, the magnitude relationship between the b wave and the d wave is reversed, compared with their magnitude relationships in cases of other belt lengths. Therefore, in the case of 165 mm, the pressure for pulse wave measurement can be regarded as being extremely low and inappropriate.

Conversely, in the more tightened cases, there are no noticeable adverse effect in particular. Therefore, in the pulse wave measurement of the present invention, pressure adjustment is performed such that subcritical points at which apparently abnormal values appear would be excluded.

Specifically, according to the control of the present invention, the average value of the voltage level of a digital signal that is A/D-converted from a measured pulse wave is compared with a previously defined predetermined range (critical point). In a case where the average value is less than the predetermined range, the measurement is determined to be inappropriate, to instruct pressure adjustment for strengthening the pressing force on the pulse wave sensor.

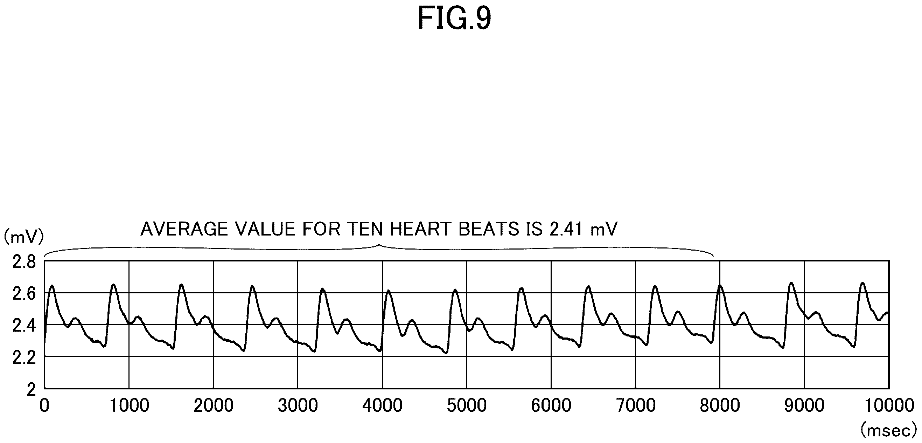

For example, FIG. 9 indicates a diagram illustrating an average value of a pulse wave output level. The average value of the voltage level of a digital signal is, for example, the average value for a predetermined number of heart beats (e.g., ten heart beats (ten cycles)), or the average value for a predetermined period (e.g., 5 seconds, 10 seconds, and the like). The present example is an example in which the average value of the output level for ten heart beats, which are the predetermined period, is 2.41 mV.

Here, the voltage level predetermined threshold (critical point) with which comparison is made to determine whether or not the pressure is appropriate is set with respect to the maximum output level of the A/D conversion circuit 133 included in the output part α. For example, the critical point, which is a previously set range, is equal to or greater than 35%, and more preferably equal to or greater than 40% of the maximum output level of the output part α. The maximum output level that is the upper limit of the output part α differs depending on the size of the strain generating body or the like, but is known beforehand.

For example, when the maximum output level of the output part α is 5 mV, the output level average value of 2.41 mV in FIG. 9 corresponds to 48% of that value, and is thus in the appropriate range.

On the other hand, for example, when the maximum output level of the output part α is 10 mV, the output level average value of 2.41 mV in FIG. 9 corresponds to 24% of that value. Therefore, the voltage level is less than the previously set range. Since the pressure on the pulse wave sensor 10 is insufficient, an instruction for strengthening the pressure, i.e., tightly fastening the belt 80 is issued.

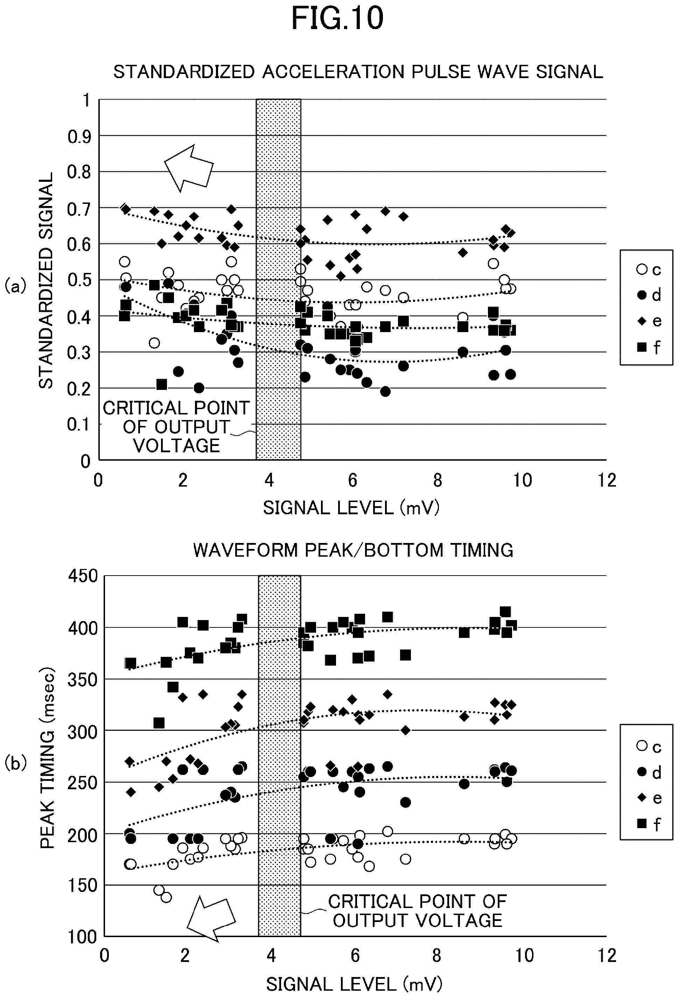

Here, FIG. 10 illustrates a diagram indicating inflection points of acceleration pulse waves corresponding to voltage values of average output levels. This example is an example in which the maximum output level is 10 mV, and the critical point that is the previously set range is 4 mV.

In the foregoing FIG. 8, the belt length was changed with respect to one subject. FIG. 10 plots the inflection points of acceleration pulse waves obtained by calculating the second derivatives of pulse waves acquired by the pulse wave measurement device 1 in a case of changing the belt length five times for two subjects.

Standardized signals are obtained by setting the maximum values of the calculated acceleration pulse waves to 1 (the values of the a wave) and setting the minimum values thereof to 0 (the values of the b wave), and the values of the c wave, the d wave, the e wave, and the f wave of the standardized signals are plotted with respect to the signal levels of the respective pressure pulse waves on the horizontal axis. The result is as illustrated in FIG. 10 (a). Specifically, FIG. 10 (a) illustrates a state resulting from resampling at 50 Hz, pulse waves acquired at a sampling frequency of 1 kHz in the form of times (timings) at which peaks (c and e) and bottoms (b, d, and f) of such standardized average acceleration pulse waves as illustrated in FIG. 8 appeared. For example, in FIG. 10 (a), the term 0-second point (origin) means a point that is on the left side of the a point by 33.3 msec.

According to FIG. 10 (a), it can be seen that among the standardized signal values of particularly the d wave, the wave height values at equal to or lower than the critical value of 4.0 mV are dispersed at higher points (to have larger values) than other values.

FIG. 10 (B) illustrates the timings at which the inflection points c, d, e, and f illustrated in FIG. 8 appeared (hereinafter, may be referred to as peak/bottom timings).

According to FIG. 10 (b), it can be seen that the peak/bottom timings are dispersed in the downward direction at equal to or lower than the critical value of 4.0 mV, i.e., that time reductions and time shifts occurred.

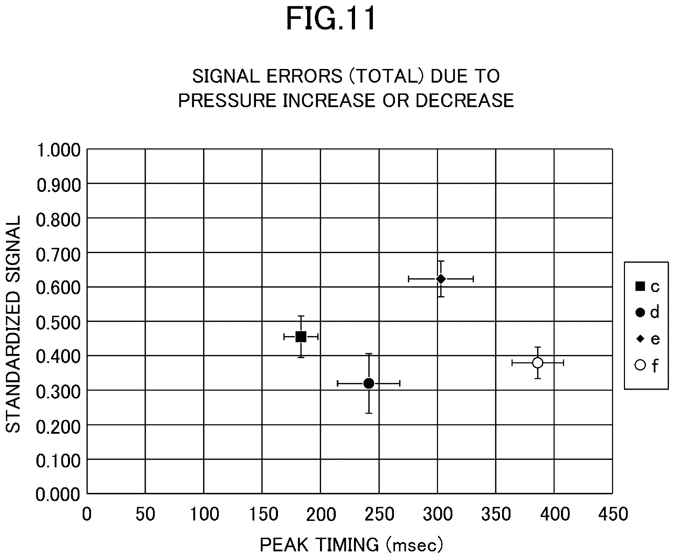

FIG. 11 is a diagram summing the dispersion of the signals due to pressing force changes within a pulse wave measurement range. Specifically, it indicates dispersion of the signals due to pressing force changes within the pulse wave measurement range, and the error bars are σ on both the vertical and horizontal axes.

As illustrated in FIG. 11, without adjustment, the dispersion of each waveform index (inflection point) of an acceleration pulse wave calculated from a biological signal, which is a pulse wave acquired from a typical pulse wave measurement device (vital sensor) is approximately around 10%.

Thus, as described above, by comparing the voltage average value, for a predetermined period, of a pulse wave signal as is with the critical point, and allowing only such critical and supercritical points as indicated on the right-hand side of the graphs of FIG. 10 (a) and (b) to move on to the main measurement of the next step, it is possible to reduce errors to the level of some percentage. As a result, the a wave, the b wave, the c wave, the d wave, the e wave, and the f wave, which are the inflection points, will appear within appropriate intensity ranges on an acceleration pulse wave that is calculated from a digital signal and used for an analysis.

In this way, by controlling the output signal level of the sensor as pressure adjustment when the pulse wave measurement device 1 is attached, it is possible to measure the blood flow or the reflected wave speed on the same or similar condition at every measurement. This restricts the dispersion of the measured values due to the pressure difference, and makes it possible to improve the accuracy of the analysis for health management or disease sign sensing using an acceleration pulse wave calculated from a pulse wave.

### (Pressure adjustment for pulse wave measurement and main measurement)

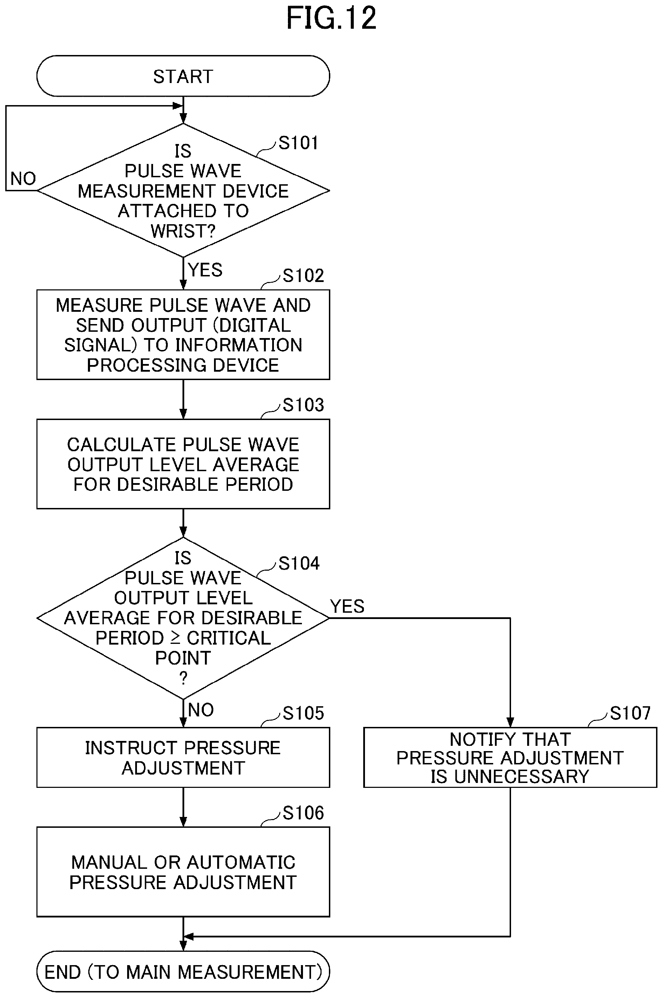

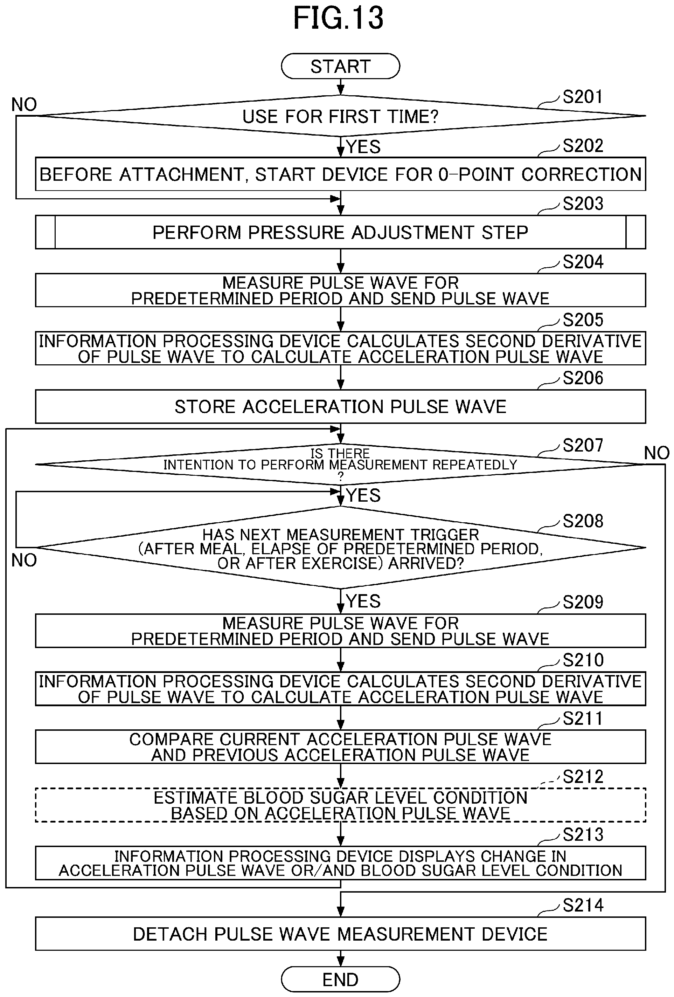

With reference to FIGS. 12 and 13, preliminary pressure adjustment for pulse wave measurement according to the present invention will be described. FIG. 12 is a detailed flowchart of a method of pressure adjustment for pulse wave measurement.

In the step S101, the user attaches the pulse wave measurement device 1 to a wrist, intending to start the measurement. As a result, before the measurement starts, pressure adjustment starts.

In the step S102, the pulse wave measurement device 1 measures a pulse wave, and sends a digital signal, which is an output, to the information processing device 5.

In the step S103, the information processing device 5 calculates the average value, for a desirable period, of the output level of the digital signal of the pulse wave. The desirable period for calculating the average value is a previously set period such as ten heart beats, 5 seconds, or the like.

In the step S104, the information processing device 5 performs comparison to determine whether the average value of the output level of the pulse wave for the desirable period is equal to or greater than the critical point.

In a case of No in S104, i.e., in a case where the average value of the output level is less than the critical point, in the step S105, the information processing device 5 instructs pressure adjustment via the display part 54 or the sound output part 55.

In the step S106, the pressure is adjusted by adjusting the fastening force of the belt 80, manually in the present embodiment. When the adjustment is completed, this flow ends, to move to the main measurement step (S204 in FIG. 13).

On the other hand, in a case where the average value of the output level of the pulse wave for the desirable period is equal to or greater than the critical point in step S104, the flow goes to S107, to notify via the display part 54 or the sound output part 55 that pressure adjustment is unnecessary. Then, the flow ends, to move to the main measurement step (S204 in FIG. 13).

In the present control, the critical point is previously set in accordance with the maximum voltage of the output part α. Therefore, it is unnecessary to adjust the pressure applied to the sensor stepwise while seeing how it goes, and it is possible to adjust the pressing force to appropriate one while shortening the time taken for the adjustment. As a result, it is possible to reduce errors.

Next, an overall flow of the pulse wave measurement including the pressure adjustment described above will be described with reference to FIG. 13. FIG. 13 is an overall flowchart of the pulse wave measurement.

When the pulse wave measurement device 1 is used for the first time after the purchase (step S201), the flow moves to the step S202 to perform 0-point correction of the pulse wave measurement device 1. In the 0-point correction, in order to adjust initial errors due to individual differences unique to the strain gauges 11a to 11d themselves or the like, before attachment, a signal is caused to be output in a state in which the detection surface of the pulse wave sensor 10 is floating, to perform adjustment such that 0 is indicated. In a case where the pulse wave measurement device 1 of the present invention is lent to a hospital, a gym, or the like, the subject starts the flow from the step S203 because the administrator has completed the 0-point adjustment step previously.

After the 0-point correction is completed, the flow moves to the step S203, to move to the pressure adjustment step for pulse wave measurement illustrated in FIG. 12.

On the other hand, in a case where the current attachment is not the first attachment (No in S201), and in a case where the device that is in an unattached state is attached to the subject again, a pulse wave is measured in the step S203, and a digital signal, which is an output, is sent to the information processing device 5.

After the preliminary pressure adjustment (S102 to S107) of FIG. 12 described above is performed in the step S203, the flow moves to the step S204, in which the pulse wave measurement device 1 measures a pulse wave for a predetermined period, and sends it to the information processing device 5. The step S204 is the main measurement, which thus may be longer than the measurement period of the adjustment described above (S102 of FIG. 12), or may be approximately the same period.

In the step S205, the information processing device 5 calculates an acceleration pulse wave by performing signal processing of the pulse wave, which is a digital signal, such as calculating the second derivative thereof and the like.

In the step S206, the information processing device 5 stores the acceleration pulse wave calculated in S205.

If there is an intention to perform the measurement repeatedly in the step S207, the subject keeps the pulse wave measurement device 1 in the attached state without unfastening the belt 80.

When the next trigger has arrived in the step S208, the flow moves to the step S209, in which the pulse wave measurement device 1 again measures a pulse wave for a predetermined period and sends it to the information processing device 5. The next measurement trigger is the opportune timing for the next measurement, such as after a meal, elapse of a predetermined period (e.g., exactly every hour, or after twelve hours), after exercise, or the like. Alternatively, the next trigger encompasses the subject's own decision to perform measurement.

Next, in the step S210, the information processing device 5 calculates an acceleration pulse wave by performing signal processing of the pulse wave, which is a digital signal, such as calculating the second derivative thereof and the like.

In the step S211, the information processing device 5 compares the acceleration pulse wave calculated in S205 and the acceleration pulse wave calculated in the step S210, to detect a change.

In the step S212, as needed, the blood sugar level condition is estimated based on the change in the acceleration pulse wave. For example, since it is known that the amplitudes and timings of inflection points on acceleration pulse waves are correlated with the blood sugar level, as needed, the blood sugar level condition is estimated based on the correlation between the waveforms of the acceleration pulse waves stored and the blood sugar level.

In the step S213, the information processing device 5 displays the result of the analysis/evaluation based on the change in the acceleration pulse wave or the result of analyzing or evaluating the blood sugar level condition.

By this control, the present invention performs pulse wave measurement as the main measurement after performing the pressure adjustment using an output level. That is, the preliminary pressure adjustment step in a case of the output level being outside the range does not shift to the main measurement in the first place. Therefore, there is no concern that the average value of the signal level (digital signal) of a pulse wave that is measured in the main measurement will be as low as the value on the right-hand side of FIG. 5 where the belt length is longer. Since the actual measurement is started when the average value of the output level for the predetermined period becomes equal to or higher than the critical point, subcritical points at which errors are likely to occur are excluded from measurement targets, resulting in that the pulse wave that is measured in the main measurement is in an adequate range. Therefore, errors can be significantly reduced.

In this way, by controlling the output signal level of the sensor to be equal to or higher than the critical point as the pressure adjustment when the pulse wave measurement device 1 is attached, it is possible to measure a pulse wave under the same or similar conditions at every measurement. Therefore, it is possible to suppress the dispersion of measured values due to pressure differences and to improve the analysis accuracy of acceleration pulse waves or the blood sugar level.

### <Configuration of the pulse wave sensor>

Next, with reference to FIGS. 14 and 15, the detailed configuration of the pulse wave sensor 10 will be described. FIG. 14 is a front view of the pulse wave sensor 10 seen from the pressure detection surface side. FIG. 15 is a cross-sectional view of the pulse wave sensor 10. Referring to FIGS. 14 and 15, the pulse wave sensor 10 includes the housing 100, the strain generating body 12, and the strain gauges 11a to 11d.

The strain generating body 12 includes a base portion 121, a beam portion 122, a load portion 123, and extension portions 124. The strain generating body 12 has a flat plate shape, and the respective components are integrally formed by, for example, a pressing process and the like. The strain generating body 12 is, for example, four-fold symmetric in a plan view. The thickness t of the strain generating body 12 excluding the load portion 123 is constant. A suitable range of the thickness t will be described later.

In FIG. 15 illustrating a state that is upside down from FIG. 2, for convenience, in the pulse wave sensor 10, the side where the load portion 123 of the strain generating body 12 is provided is referred to as the upper side or one side, and the side where the load portion 23 is not provided is referred to as the lower side or the other side. A surface of each part on the side where the load portion 23 is provided is referred to as one surface or the upper surface, and the other surface of each part on the side where the load portion 123 is not provided is referred to as the other surface or the lower surface. However, the pulse wave sensor 10 can be used in an inverted state or arranged at any angle. The plan view refers to the object viewed in the direction normal to the upper surface of the strain generating body 12, and the planar shape refers to the shape of the object viewed in the direction normal to the upper surface of the strain generating body 12.

In the pulse wave sensor 10, the housing 100 holds the strain generating body 12. The housing 100 has a hollow cylindrical shape, and the one surface side is closed and the other surface (the surface on the detection side) is opened. The housing 100 can be formed of, for example, metal, resin, or the like. The substantially disk-shaped strain generating body 12 is fixed by an adhesive or the like so as to close an opening on the upper surface side of the housing 100.

In the strain generating body 12, the base portion 121 is a circular frame-shaped (ring-shaped) region outside the circular dashed line illustrated in FIG. 14. The region inside the circular dashed line may sometimes be referred to as the circular opening. That is, the base portion 121 of the strain generating body 12 has the circular opening. The width w₁ of the base portion 121 is, for example, in a range from 1 mm to 5 mm. A suitable range of the inner diameter d (that is, the diameter of the circular opening) of the base portion 121 will be described later.

The beam portion 122 is provided so as to bridge the inside of the base portion 121. The beam portion 122 has, for example, two beams that cross each other in a cross shape in a plan view, and the region where the two beams cross includes a center of the circular opening. In the example of FIG. 14, one beam that forms a cross has its longitudinal direction in the X-direction, and the other beam that forms a cross has its longitudinal direction in the Y direction, and they are orthogonal to each other. Each of the two orthogonal beams is disposed inward relative to the inner diameter d (diameter of the circular opening) of the base portion 121 and is preferably as long as possible. That is, the length of each beam is preferably approximately equal to the diameter of the circular opening. In each beam constituting the beam portion 122, the width w₂ other than the region where the two beams cross is constant, for example, in a range from 1 mm to 5 mm. The width w₂ is not necessarily constant. However, making the width w₂ constant is preferable because this makes it possible to detect the strain linearly.

The load portion 123 is provided on the beam portion 122. The load portion 123 is provided, for example, on a region where two beams constituting the beam portion 122 cross. The load portion 123 projects from the upper surface of the beam portion 122. The amount of projection of the load portion 123 relative to the upper surface of the beam portion 122 is, for example, approximately 0.1 mm. The beam portion 122 is flexible and elastically deformed when a load is applied to the load portion 123.

The four extension portions 124 are sectorial parts extending in the direction of the beam portion 122 from the inside of the base portion 121 in a plan view. A gap of approximately 1 mm is provided between each extension portion 124 and the beam portion 122. Since the extension portions 124 do not contribute to the sensing of the pulse wave sensor 10, they do not need to be provided.

As illustrated in FIG. 15, the control board 17 is provided in the pulse wave sensor 10. The control board 17 is, for example, a flexible substrate, and is mounted with the AFE 13, the controller 14, the communication part 15, the battery 16, and the like illustrated in FIG. 4. The control board 17 is connected to the strain gauges 11a to 11d through a cable (not illustrated) through which an electric signal is input or output. The cable is, for example, a wire rod such as a shield cable. The control board 17 may be provided outside the housing 100 because it only needs to be provided inside the pulse wave measurement device 1.

The strain gauges 11a to 11d are provided on the strain generating body 12. The strain gauges 11a to 11d can be provided, for example, on the lower surface side of the beam portion 122. Since the beam portion 122 is a flat plate shape, the strain gauges can be easily attached to the beam portion 122. One or more strain gauges 11a to 11d may be provided, but in the present embodiment, four strain gauges 11a to 11d are provided. By providing four strain gauges 11a to11d, strain can be detected by a full bridge.

Two strain gauges 11b and 11d of the four strain gauges are positioned on the beam having its longitudinal direction in the X-direction, at a portion of the beam close to the load portion 123 (at a portion on the center side of the circular opening), so that the two of the four strain gauges face each other across the load portion 23 in the plan view. The other two strain gauges 11a and 11c of the four strain gauges are positioned on the beam having its longitudinal direction in the Y-direction, at a portion of the beam close to the base portion 121, so that the other two of the four strain gauges face each other across the load portion 23 in the plan view. With such an arrangement, compressive and tensile forces can be effectively detected and a large output can be obtained by the full bridge.

The pulse wave sensor 10 is used while being fixed to an arm of a subject so that the load portion 123 is disposed above the radial artery of the subject. When a load is applied to the load portion 123 in response to a pulse wave of the subject and the beam portion 122 is elastically deformed, the resistance values of the resistors of the strain gauges 11a to 11d change. The pulse wave sensor 10 can detect a pulse wave based on the change of the resistance values of the resistors of the strain gauges 11a to 11d caused by the deformation of the beam portion 122. The pulse wave is, for example, output as periodic voltage changes from the AFE 13, which is a measuring circuit connected with electrodes of the strain gauges 11a to 11d.

### [Configuration of the strain gauges]

Here, the configuration of the strain gauges will be described with reference to FIGS. 16 and 17. FIG. 16 is a plan view illustrating the strain gauge 11a according to one embodiment of the present invention. FIG. 17 is a cross-sectional view illustrating the strain gauge 11a according to one embodiment, and illustrates a cross-section of FIG. 16 along a line A-A. The configuration of, for example, the strain gauge 11a will be described below. However, the strain gauges 11b, 11c, and 11d have the same configuration.

With reference to FIGS. 16 and 17, the strain gauge 11a includes a substrate 110, a resistor 111, wirings 112 and 113, electrodes 114 and 115, and a cover layer 116. For convenience, FIG. 16 only illustrates the contour of the cover layer 116 by a broken line. The cover layer 116 may be provided as needed.

The strain gauge 11a illustrated in FIG. 16 has a side of its substrate 110, on which no components are provided, pasted on the beam portion 122 extending in the X direction illustrated in FIG. 14. Here, the side of the substrate 110, on which no components are provided, is pasted on the lower surface (back surface) of the beam portion 122 by an adhesive or the like. A plan view of FIG. 16 refers to the object viewed in the direction normal to the upper surface 110U of the substrate 110 from the upper side to the lower side. A planar shape refers to the shape of the object viewed in the normal direction.

The substrate 110 is a member that serves as a base layer for forming the resistor 111 and the like, and the substrate 110 has flexibility. The thickness of the substrate 110 is not particularly limited and can be, for example, approximately in a range from 5 µm to 500 µm. Considering the strain transmission property from the outer surface of the strain generating body 12 to a sensitive part and dimensional stability against environmental changes, the thickness of the substrate 110 is preferably in a range from 5 µm to 200 µm. Considering the insulating property, the thickness of the substrate 110 is preferably 10 µm or greater.

The substrate 110 is formed from, for example, an insulating resin film such as a polyimide (PI) resin, an epoxy resin, a polyetheretherketone (PEEK) resin, a polyethylene naphthalate (PEN) resin, a polyethylene terephthalate (PET) resin, a polyphenylene sulfide (PPS) resin, a liquid crystal polymer (LCP) resin, a polyolefin resin, and the like. The film refers to a member having a thickness of approximately 500 µm or less and having flexibility.

When the substrate 110 is formed from an insulating resin film, the insulating resin film may contain fillers, impurities or the like. The substrate 110 may be formed from, for example, an insulating resin film containing a filler such as silica, alumina, or the like.

Materials other than the resin for the substrate 110 include, for example, crystalline materials such as SiO₂, ZrO₂ (including YSZ), Si, Si₂N₃, Al₂O₃ (including sapphire), ZnO, perovskite-based ceramics (CaTiO₃, BaTiO₃), or the like. In addition to the crystalline materials, amorphous glass or the like may be used as the material of the substrate 110. Metals such as aluminum, aluminum alloy (duralumin), titanium, or the like may be used as materials for the substrate 110. When using a metal, an insulating film is formed on the substrate 110 made of metal.

The resistor 111 is a thin film formed in a predetermined pattern on the upper side of the substrate 110. In the strain gauge 11a, the resistor 111 is a sensitive part that causes a resistance change when being strained. The resistor 111 may be formed directly on the upper surface 110U of the substrate 110, or through other layers (a functional layer 117 illustrated in FIG. 17) on the upper surface 110U of the substrate 110. In FIG. 16, the resistor 111 is illustrated in a dark-colored pear-skin pattern for convenience.

The resistor 111 has a structure in which a plurality of thin and long portions are arranged at predetermined intervals with the longitudinal direction set in the same direction (the horizontal direction in the example of FIG. 16), and the end portions of adjacent thin and long portions are connected on the alternate sides, to have a zigzag-folded-back shape as a whole. The longitudinal direction of the plurality of thin and long portions is a grid direction, and the direction perpendicular to the grid direction is a grid width direction (the vertical direction in the example of FIG. 16).

In the resistor 111, the left end portion of the thin and long portion that is positioned on the uppermost side in FIG. 16 is bent in the upward direction to reach an end 122e₁ of the resistor 111 on one side in the grid width direction. The left end portion of the thin and long portion that is positioned on the lowermost side in FIG. 16 is bent in the downward direction to reach an end 122e₂ of the resistor 111 on the other side in the grid direction. The respective ends 122e₁ and 122e₂ are electrically connected to the electrodes 114 and 115 through the wirings 112 and 113. In other words, the wirings 112 and 113 electrically connect the ends 122e₁ and 122e₂ of the resistor 111 in the grid width direction to the electrodes 114 and 115.

The resistor 111 can be formed, for example, from a material containing chromium (Cr), a material containing nickel (Ni), or a material containing both Cr and Ni. That is, the resistor 111 can be formed from a material containing at least one of Cr or Ni. The material containing Cr includes, for example, a Cr mixed phase film. The material containing Ni includes, for example, copper nickel (Cu-Ni). The material containing both Cr and Ni includes, for example, nickel chromium (Ni-Cr).

Here, the Cr mixed phase film is a film in which Cr, CrN, Cr₂N, and the like are mixed. The Cr mixed phase film may contain unavoidable impurities such as chromium oxide and the like.

The thickness of the resistor 111 is not particularly limited and can be, for example, approximately in a range from 0.05 µm to 2 µm. In particular, when the thickness of the resistor 111 is 0.1 µm or more, the crystallinity of the crystals constituting the resistor 111 (for example, the crystallinity of α-Cr) is improved. When the thickness of the resistor 111 is 1 µm or less, (i) film cracks and (ii) warpage of the film from the substrate 110, which are caused by the internal stress of the film constituting the resistor 111 can be reduced.

Taking into consideration making lateral sensitivity less likely to occur and the wire breakage countermeasures, the width of the resistor 111 is preferably 10 µm or greater and 100 µm or less. To be more specific, the width of the resistor 111 is preferably 10 µm or greater and 70 µm or less, and more preferably 10 µm or greater and 50 µm or less.

For example, when the resistor 111 is a Cr mixed phase film, the stability of the gauge characteristic can be improved by using alpha chromium (α-Cr), which is a stable crystalline phase, as the main component. Moreover, when the resistor 111 is a Cr mixed phase film, using α-Cr as the main component of the resistor 111 can bring the gauge rate of the strain gauge 11a to 10 or more, and the temperature coefficient of strain gauge rate TCS and the temperature coefficient of resistance TCR to within a range from -1,000 ppm/°C to +1,000 ppm/°C. Here, term "the main component" means that the component occupies 50% by weight or more of the all materials constituting the resistor. From the viewpoint of improving the gauge characteristics, the resistor 111 preferably contains 80% by weight or more of α-Cr. Moreover, from the same viewpoint, the resistor 111 more preferably contains 90% by weight or more of α-Cr. α-Cr is Cr having a bcc structure (body-centered cubic lattice structure).

When the resistor 111 is a Cr mixed phase film, CrN and Cr₂N contained in the Cr mixed phase film are preferably 20% by weight or less. When CrN and Cr₂N contained in the Cr mixed phase film are 20% by weight or less, the lowering of the gauge rate of the strain gauge 11a can be suppressed.

As the ratio of CrN and Cr₂N in the Cr mixed phase film, the proportion of Cr₂N is preferably 80% by weight or more and less than 90% by weight relative to the total weight of CrN and Cr₂N. More specifically, as the ratio, the proportion of Cr₂N is more preferably 90% by weight or more and less than 95% by weight relative to the total weight of CrN and Cr₂N. Cr₂N has semiconducting properties. Therefore, when the proportion of Cr₂N mentioned above is 90% by weight or more and less than 95% by weight, the decrease in TCR (negative TCR) becomes more remarkable. Moreover, when the proportion of Cr₂N mentioned above is 90% by weight or more and less than 95% by weight, ceramization of the resistor 111 can be reduced. Therefore, it is possible to make brittle fracture of the resistor 111 less likely to occur.

Meanwhile, CrN has an advantage of being chemically stable. Including more CrN in the Cr mixed phase film can reduce the possibility of occurrence of unstable N. Therefore, a stable strain gauge can be obtained. Here, the term "unstable N" means trace N₂ or atomic N that may be present in the Cr mixed phase film. These types of unstable N may slip out of the film depending on the external environment (e.g., a high-temperature environment). When unstable N slips out of the film, film stress in the Cr mixed phase film may change.

The wirings 112 and 113 are provided on the substrate 110. The wirings 112 and 113 are electrically connected to the resistor 111 and the electrodes 114 and 115. The wirings 112 and 113 are not limited to a straight line shape, and may be any desirable pattern. The wirings 112 and 113 may have a desirable width and a desirably selected length. For convenience, FIG. 16 illustrates the wirings 112 and 113 in a lighter-colored pear-skin pattern than the resistor 111.

The electrodes 114 and 115 are provided on the substrate 110. The electrodes 114 and 115 are electrically connected to the resistor 111 through the wirings 112 and 113. In a plan view, the electrodes 114 and 115 are formed in a substantially rectangular shape wider than the wirings 112 and 113. The electrodes 114 and 115 are a pair of electrodes for outputting a change in the resistance value of the resistor 111 caused by strain to the outside. For example, a lead wire (not illustrated) for external connection is joined to the electrodes 114 and 115. A metal layer having a low resistance such as copper or the like, or a metal layer having a good soldering property such as gold or the like may be laminated on the upper surface of the electrodes 114 and 115. Although the resistor 111, the wirings 112 and 113, and the electrodes 114 and 115 are denoted by different numerals for convenience, they may be formed integrally from the same material in the same step. For convenience, FIG. 16 illustrates the electrodes 114 and 115 in the same pear-skin pattern as the wirings 112 and 113.

The cover layer 116 is provided on the substrate 110 as needed. The cover layer 116 is provided on the upper surface 110U of the substrate 110 so as to cover the resistor 111 and the wirings 112 and 113 and to expose the electrodes 114 and 115. Examples of the material of the cover layer 116 include an insulating resin such as a PI resin, an epoxy resin, a PEEK resin, a PEN resin, a PET resin, a PPS resin, or a composite resin (for example, silicone resin, polyolefin resin). The cover layer 116 may contain fillers or pigments. The thickness of the cover layer 116 may be, for example, approximately in a range from 2 µm to 30 µm. By providing the cover layer 116, it is possible to inhibit occurrence of mechanical damage or the like to the resistor 111. Moreover, by providing the cover layer 116, it is possible to protect the resistor 111 from moisture or the like.

In a case where a Cr mixed phase film is used as the material of the resistor 111 of the strain gauge 11a, sensitivity enhancement and size reduction can be realized. For example, in a case where a Cr mixed phase film is used as the material of the resistor 111, it is possible to obtain an output of 0.3 mV/2 V or higher, compared with an output of an existing strain gauge of approximately 0.04 mV/2 V. Moreover, in a case where a Cr mixed phase film is used as the material of the resistor 111, the size (gauge length × gauge width) can be reduced to 0.3 mm × 0.3 mm, compared with the size (gauge length × gauge width) of an existing strain gauge of approximately 3 mm × 3 mm.

Therefore, the strain gauge 11a in which a Cr mixed phase film is used as the material of the resistor 111 is suitable for use in the pulse wave sensor 10 in the pulse wave measurement device 1, which is a wearable device that can be retrofitted on even a small space of the strain generating body 12. Moreover, the strain gauge 11a in which a Cr mixed phase film is used as the material of the resistor 111 has a higher resistance than that of an existing strain gauge. Therefore, in a case of driving the strain gauge 11a by an electric cell as the battery 16, it is possible to save power consumption. As a result, it is possible to prolong the battery life and to measure a pulse wave of a subject for a longer term or repeatedly.

The functional layer 117 may be patterned into a planar shape that is approximately the same as the planar shape of, for example, the resistor 111, the wirings 112 and 113, and the electrodes 114 and 115. In the configuration of FIG. 17, by providing the functional layer 117 under the metal layer in which the components such as the wirings are to be formed, it is possible to promote crystal growth of the metal layer, and to produce a metal layer made of a stable crystal phase. As a result, the stability of the gauge characteristics of the strain gauge 11a is improved. Moreover, diffusion of a material, which is a constituent of the functional layer, into the metal layer improves the gauge characteristics of the strain gauge 11a.

### (Another lamination structure of the strain gauge)

FIG. 17 illustrates a cross-sectional shape in a case of providing the functional layer 117 as an underlayer of the resistor 111, the wirings 112 and 113, and the electrodes 114 and 115. However, the functional layer 117 does not need to be provided.

### <Embodiment 2>

In the configuration described above, the pulse wave measurement device adjusts the pressing force of the pulse wave sensor on the skin by adjusting the length of the belt 80. The pressing force may be adjusted by any other configuration.

With reference to FIG. 18, a modified example of the pulse wave measurement device will be described. FIG. 18 is a cross-sectional view of a head member H of a pulse wave measurement device 1A according to Embodiment 2.

As illustrated in FIG. 18, the head member H of the pulse wave measurement device 1A according to the present modified example includes, for example, a pulse wave sensor 10, a box-shaped sensor fastener 201, belt fasteners 301 and 302, and a biasing force adjusting mechanism 60. The pulse wave sensor 10 has the same configuration as Embodiment 1.

The sensor fastener 201 is a pressing part to which the side surfaces and the upper surface of the pulse wave sensor 10 are fastened, and that is configured to receive a pressing force from the biasing force adjusting mechanism 60 to press the pulse wave sensor 10 to a wrist of the user.

The biasing force adjusting mechanism 60 is an adjusting member, and includes an upper surface lifting plate 61, an annular-tube-shaped support plate 62, and a spring 63. The spring 63 is, for example, a coil spring, and is fixed to the upper surface of the sensor fastener 201. The biasing force adjusting mechanism 60 can adjust the force of the spring 63 to press the pulse wave sensor 10 in the direction N via the sensor fastener 201, by moving the upper surface lifting plate 61 upward or downward on the support plate 62.

As an example of the biasing force adjusting mechanism 60, a jagged engaging projection extending in the up-down direction is provided on the support plate 62, and the upper surface lifting plate 61 descends by an amount corresponding to being pressed by the subject's pressing and stops at the position. Therefore, in accordance with the pressing amount, the biasing force exerted by the spring 63 on the sensor fastener 201 changes. Alternatively, the upper surface lifting plate 61 may be threadedly engaged with the support plate 62, and the subject may change the biasing force by rotating the upper surface lifting plate 61 relative to the support plate 62 to thereby move the upper surface lifting plate 61 down by the amount of rotation and by stopping the upper surface lifting plate 61 at the position.

In this way, according to the present embodiment, changing the height of the upper surface lifting plate 61 can cause the spring 63 to change the biasing pressure toward the subject. Therefore, the pressure can be applied to the radial artery of the subject while being adjusted to a suitable value. As a result, the pulse wave measurement device 1A can obtain a good close contact between the subject and the pulse wave sensor 10 and improve the pulse wave measurement accuracy.

In the present embodiment, by notifying that the output signal level of the sensor should be equal to or higher than the critical point when the pulse wave measurement device 1A is attached, to invite the pressure to be controlled via the biasing force adjusting mechanism 60, it is possible to perform pulse wave measurement under the same or similar conditions at every measurement. As a result, it is possible to suppress the dispersion of measured values due to pressure differences, and to improve the accuracy for analyzing acceleration pulse waves and the blood sugar level.

### <Embodiment 3>

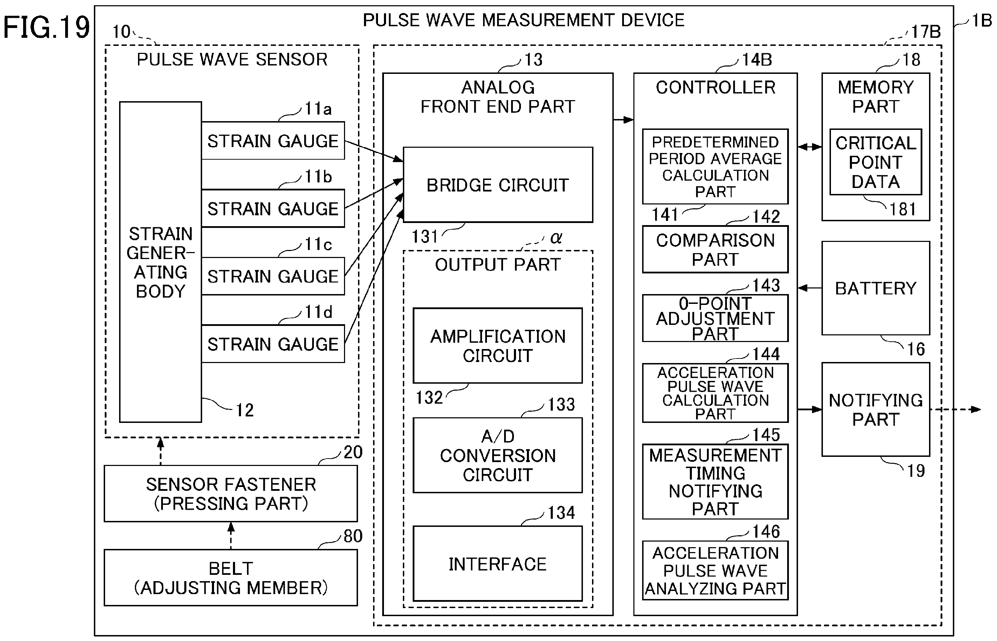

FIG. 19 is an overall block diagram of a pulse wave measurement device 1B according to Embodiment 3.

In Embodiment 1, the calculation function for comparison and the like is executed on the information processing device side. However, calculation may be performed in the pulse wave measurement device 1B, to instruct adjustment. In this case, the pulse wave measurement device does not need to communicate with the information processing device because it can execute the calculation function inside.

In the present example, a controller 14B of the pulse wave measurement device 1B includes, in an executable manner, a predetermined period average calculation part 141, a comparison part 142, a 0-point adjustment part 143, an acceleration pulse wave calculation part 144, a measurement timing notifying part 145, an acceleration pulse wave analyzing part 146, and the like. A control board 17B is provided with a memory part 18 and a notifying part 19. The notifying part 19 is composed of, for example, a sound output part, a display part, or a light emitting means or the like, and also functions as an adjustment instruction part for instructing pressure adjustment.

According to the present embodiment, in the pulse wave measurement device 1B, the voltage level of a digital signal output as a measured pulse wave is compared with a range that is previously set with respect to the maximum voltage level of the output part, and in a case where the voltage level of the digital signal is lower than the range, the notifying part 19 instructs adjustment of the pressing force on the pulse wave sensor 10. Then, the user adjusts the length of the belt 80 or the position of the upper surface lifting plate 61 of the biasing force adjusting mechanism 60.

Therefore, even when the pressing force or the like differs due to the girth of the arm that is different from subject to subject, it is possible to adjust the pressing force to appropriate one while shortening the time taken for the adjustment, and to reduce errors.

FIG. 19 illustrates a self-contained configuration of the pulse wave measurement device 1B alone, with the entirety of the calculation function provided on the pulse wave measurement device 1B side. However, this configuration may also be able to communicate with an information processing device, and some of the functions of the controller 14B of FIG. 19, e.g., the functions of the acceleration pulse wave calculation part 144 and the acceleration pulse wave analyzing part 146 involving a large amount of processing may be provided on the information processing device side.

### <Embodiment 4>

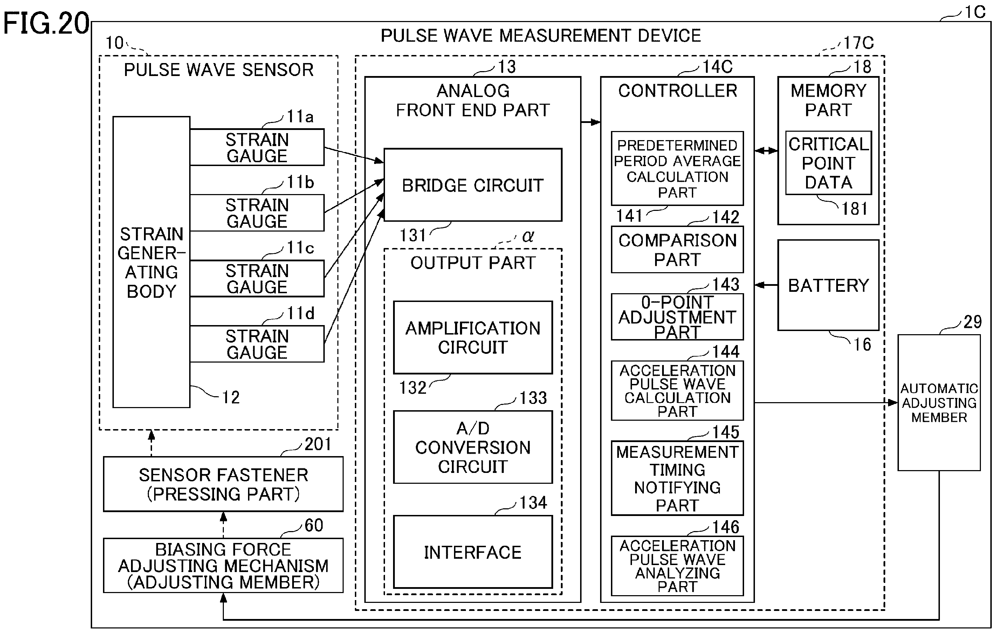

FIG. 20 is an overall block diagram of a pulse wave measurement device 1C according to Embodiment 4. In the present embodiment, the pulse wave measurement device 1C includes an automatic adjusting member 29, and has an automatic adjusting function. Therefore, calculations are performed in a controller 14C of the pulse wave measurement device 1C, to perform adjustment. Since the present embodiment premises automatic adjustment, a configuration that can adjust the pressure by a biasing force adjusting mechanism 60 such as that of FIG. 18 is preferable.

According to the present embodiment, in the pulse wave measurement device 1C, the voltage level of a digital signal output as a measured pulse wave is compared with a range that is previously set with respect to the maximum voltage level of the output part α, and in a case where the voltage level of the digital signal is lower than the range, the pressing force on the pulse wave sensor 10 is automatically adjusted. For example, as the automatic adjustment, the upper surface lifting plate 61 is automatically moved up or down, to adjust the pressure automatically.

Therefore, even when the pressing force or the like differs due to the girth of the arm that is different from subject to subject, it is possible to adjust the pressing force to appropriate one while shortening the time taken for the adjustment, and to reduce errors.

Although preferable embodiments and the like have been described in detail above, the embodiments and the like described above are non-limiting, and various modifications and replacements are applicable to the embodiments and the like described above without departing from the scope described in the claims.

This international application claims priority to Japanese Patent Application No. 2022-106687, filed June 30, 2022. The entire contents of Japanese Patent Application No. 2022-106687 are incorporated herein by reference.

### REFERENCE SIGNS LIST

1, 1A, 1B, 1C: pulse wave measurement device, 5: information processing device, 10: pulse wave sensor, 11a, 11b, 11c, 11d: strain gauge, 12: strain generating body, 13: AFE, 20, 201: sensor fastener (pressing part), 60: biasing force adjusting mechanism (adjusting member), 80: belt (adjusting member), 131: bridge circuit, 132: amplification circuit, 133: A/D conversion circuit (A/D converter), 1000: pulse wave measurement system, α: output part

## Claims

1. A method of adjusting a pulse wave measurement device attachable to a wrist of a user, wherein the pulse wave measurement device includes:
a pulse wave sensor including a strain gauge and configured to acquire a pulse wave by measuring a pulse of a radial artery;
a pressing part configured to press the pulse wave sensor to the radial artery; and
an output part configured to output a measured value of the pulse wave sensor as a digital signal,
the method, comprising,
acquiring a pulse wave using the pulse wave sensor when the pulse wave measurement device is attached to a wrist of a user, and outputting a measured value thus acquired, as a digital signal;
comparing a voltage level of the digital signal with a range that is previously set with respect to a maximum voltage level of the output part; and
adjusting or instructing a pressing force on the pulse wave sensor, in a case where the voltage level of the digital signal is outside the range that is previously set.

2. The method of adjusting the pulse wave measurement device according to claim 1,
wherein the range of the voltage level that is previously set and with which comparison is made in the comparing is equal to or higher than 35% of a maximum output level of the output part.

3. The method of adjusting the pulse wave measurement device according to claim 1,
wherein an output level of the digital signal that is subjected to comparison in the comparing is an average voltage of the digital signal for a desirable period.

4. The method of adjusting the pulse wave measurement device according to claim 1, comprising:
performing 0-point correction of the digital signal acquired by the pulse wave sensor as an initial adjustment, before the comparing, when the pulse wave measurement device is attached to the wrist of the user for a first time.

5. A pulse wave measurement system, comprising:
a pulse wave measurement device attachable to a wrist of a user; and
an information processing device capable of communicating with the pulse wave measurement device,
wherein the pulse wave measurement device includes:
a pulse wave sensor including a strain gauge and configured to acquire a pulse wave of a radial artery when the pulse wave measurement device is attached to the wrist of the user;
an output part configured to output a measured value of the pulse wave sensor as a digital signal;
a pressing part configured to press the pulse wave sensor to the radial artery; and
an adjusting member configured to adjust a pressing force on the pulse wave sensor, and
the information processing device includes:
a comparison part configured to compare a voltage level of the digital signal with a range that is previously set with respect to a maximum voltage level of the output part; and
an adjustment instruction part configured to instruct adjustment of the pressing force on the pulse wave sensor in a case where the voltage level of the digital signal is outside the range that is previously set.

6. The pulse wave measurement system according to claim 5,
wherein the pulse wave measurement device includes a belt for attachment, the belt being attached to the wrist of the user and being wound outside the pulse wave sensor,
the pulse wave sensor includes a strain generating body on which the strain gauge is positioned and that is brought into contact with the wrist of the user,
the pressing part is a sensor fastener configured to hold a side of the pulse wave sensor that is opposite to the strain generating body, and
the adjusting member is the belt, and is capable of adjusting a biasing force of the pressing part by adjusting a belt length of the belt.

7. The pulse wave measurement system according to claim 5,
wherein the pulse wave sensor includes a strain generating body on which the strain gauge is positioned and that is brought into contact with the wrist of the user,
the pressing part is a sensor fastener configured to hold a side of the pulse wave sensor that is opposite to the strain generating body, and
the adjusting member is a biasing force adjusting mechanism configured to be brought into contact with a side of the sensor fastener that is opposite to the pulse wave sensor and bias the sensor fastener toward the wrist of the user.

8. The pulse wave measurement system according to claim 5,
wherein the pulse wave sensor of the pulse wave measurement device outputs an analog signal, and
the output part includes an amplification circuit and an A/D converter.

9. The pulse wave measurement system according to claim 5,
wherein the information processing device calculates a second derivative of the digital signal that is a measured value of the pulse wave after the pressing force is adjusted, to calculate an acceleration pulse wave.

10. The pulse wave measurement system according to claim 5,
wherein the pulse wave sensor includes a strain generating body to which the strain gauge is attached, and
the pulse wave sensor is configured to detect the pulse wave based on a change in a resistance value of the strain gauge caused by deformation of the strain generating body.

11. The pulse wave measurement system according to claim 10,
wherein the strain gauge includes a strain gauge including a resistor made of a film containing Cr, CrN, and Cr₂N.

12. The pulse wave measurement system according to claim 5,
wherein the pulse wave measurement device further includes a position detector configured to detect a position of the pulse wave sensor with respect to the radial artery.

13. A pulse wave measurement device attachable to a wrist of a user, the pulse wave measurement device comprising:
a pulse wave sensor including a strain gauge and configured to acquire a pulse wave of a radial artery when the pulse wave measurement device is attached to a wrist of a user;
an output part configured to output a measured value of the pulse wave sensor as a digital signal;
a comparison part configured to compare a voltage level of the digital signal with a range that is previously set with respect to a maximum voltage level of the output part;
a pressing part configured to press the pulse wave sensor to the radial artery; and
an adjusting member configured to adjust a pressing force on the pulse wave sensor in a case where the voltage level of the digital signal is outside the range that is previously set.
